(19) Europäisches Patentamt

European Patent Office

Office européen des brevets

(11) **EP 3 042 962 A1**

(12) **EUROPEAN PATENT APPLICATION**

(43) Date of publication:
**13.07.2016 Bulletin 2016/28**

(51) Int Cl.:
***C12Q 1/68*** $^{(2006.01)}$

(21) Application number: **15150338.0**

(22) Date of filing: **07.01.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **UNIVERSITÄTSKLINIKUM ERLANGEN
91054 Erlangen (DE)**

(72) Inventors:
• **Strick, Reiner
91077 Kleinsendelbach (DE)**
• **Henke, Christine
91052 Erlangen (DE)**

(74) Representative: **Vossius & Partner
Patentanwälte Rechtsanwälte mbB
Siebertstrasse 3
81675 München (DE)**

(54) **Tag-aided sense-antisense transcript detection**

(57)    The present invention relates to a method for determining the sense/antisense polarity of an RNA transcript of interest in a sample, the method comprising: (a) reverse transcribing an RNA transcript of interest present in the sample, wherein the reverse transcription reaction comprises: (i) a primer linked to an oligonucleotide tag , wherein the primer specifically binds to (1) the sense strand of the RNA transcript of interest; or (2) the antisense strand of the RNA transcript of interest; (ii) a reverse transcriptase lacking or having a reduced RNase H activity; and (iii) an inhibitor of DNA-dependent DNA synthesis; (b) removing the RNA strand from the RNA:cDNA hybrid strand produced in step (a); (c) amplifying the first strand cDNA obtained in step (b) with a forward primer that specifically binds to said first strand cDNA and a reverse primer, wherein the reverse primer comprises or consists of a nucleic acid sequence that corresponds to the nucleotide sequence of the oligonucleotide tag, or to a portion thereof; and (d) detecting the presence of an amplification product, wherein the presence of an amplification product obtained after reverse transcribing the RNA transcript of interest in a reaction comprising the primer of (a)(i)(1) indicates that the RNA transcript is a sense-strand RNA transcript and the presence of an amplification product obtained after reverse transcribing the RNA transcript of interest in a reaction comprising the primer of (a)(i)(2) indicates that the RNA transcript is an antisense-strand RNA transcript; and wherein the oligonucleotide tag employed in step (a)(1) has a nucleic acid sequence that is not endogenously present in the genome of the organism from which the sample has been obtained. The present invention further relates to an oligonucleotide comprising or consisting of the nucleic acid sequence shown in SEQ ID NO:1 as well as a kit comprising an oligonucleotide according to the invention and at least one of: (i) a reverse transcriptase lacking or having a reduced RNase H activity; (ii) an inhibitor of DNA-dependent DNA synthesis; (iii) RNase H; and (iv) means for amplification of cDNA.

EP 3 042 962 A1

SEQUENCE LISTING

<110> Universitätsklinikum Erlangen

<120> Tag-aided sense-antisense transcript detection

<130> X3351 EP

<160> 10

<170> BiSSAP 1.2

<210> 1
<211> 23
<212> DNA
<213> Artificial Sequence

<220>
<221> source
<222> 1..23
<223> /mol_type="unassigned DNA"
      /note="Oligonucleotide tag 1"
      /organism="Artificial Sequence"

<400> 1
gcacacgacg acagacgacg cac                                                    23

<210> 2
<211> 23
<212> DNA
<213> Artificial Sequence

<220>
<221> source
<222> 1..23
<223> /mol_type="unassigned DNA"
      /note="Oligonucleotide tag 2"
      /organism="Artificial Sequence"

<400> 2
gcacagcacg acagacgacg cac                                                    23

<210> 3
<211> 23
<212> DNA
<213> Artificial Sequence

<220>
<221> source
<222> 1..23
<223> /mol_type="unassigned DNA"
      /note="Oligonucleotide tag 3"
      /organism="Artificial Sequence"

<400> 3
gcacacgacg acagagcacg cac                                                    23

<210> 4
<211> 23
<212> DNA
<213> Artificial Sequence

<220>
<221> source
<222> 1..23

<223> /mol_type="unassigned DNA"
      /note="Oligonucleotide tag 4"
      /organism="Artificial Sequence"

<400> 4
gcacacgacg acagacgacg cag                                                    23


<210> 5
<211> 23
<212> DNA
<213> Artificial Sequence

<220>
<221> source
<222> 1..23
<223> /mol_type="unassigned DNA"
      /note="Oligonucleotide tag 5"
      /organism="Artificial Sequence"

<400> 5
gcagacgacg acagacgacg cac                                                    23


<210> 6
<211> 23
<212> DNA
<213> Artificial Sequence

<220>
<221> source
<222> 1..23
<223> /mol_type="unassigned DNA"
      /note="Oligonucleotide tag 6"
      /organism="Artificial Sequence"

<400> 6
cgacacgacg acagacgacg cac                                                    23


<210> 7
<211> 21
<212> DNA
<213> Artificial Sequence

<220>
<221> source
<222> 1..21
<223> /mol_type="unassigned DNA"
      /note=" -actin sense gene and strand specific primer"
      /organism="Artificial Sequence"

<400> 7
gctggtcgtc gacaacggct c                                                      21


<210> 8
<211> 25
<212> DNA
<213> Artificial Sequence

<220>
<221> source
<222> 1..25
<223> /mol_type="unassigned DNA"
      /note=" -actin antisense gene and strand specific primer"
      /organism="Artificial Sequence"

```
<400> 8
caaacatgat ctgggtcatc ttttc                                          25


<210> 9
<211> 28
<212> DNA
<213> Artificial Sequence

<220>
<221> source
<222> 1..28
<223> /mol_type="unassigned DNA"
      /note="Mart6 sense gene and strand specific primer"
      /organism="Artificial Sequence"

<400> 9
atggtccaac ctcggacctc caaaactg                                       28


<210> 10
<211> 28
<212> DNA
<213> Artificial Sequence

<220>
<221> source
<222> 1..28
<223> /mol_type="unassigned DNA"
      /note="Mart6 antisense gene and strand specific primer"
      /organism="Artificial Sequence"

<400> 10
ataccaggaa ggccactctc tcggcttc                                       28
```

**Description**

The present invention relates to a method for determining the sense/antisense polarity of an RNA transcript of interest in a sample, the method comprising: (a) reverse transcribing an RNA transcript of interest present in the sample, wherein the reverse transcription reaction comprises: (i) a primer linked to an oligonucleotide tag , wherein the primer specifically binds to (1) the sense strand of the RNA transcript of interest; or (2) the antisense strand of the RNA transcript of interest; (ii) a reverse transcriptase lacking or having a reduced RNase H activity; and (iii) an inhibitor of DNA-dependent DNA synthesis; (b) removing the RNA strand from the RNA:cDNA hybrid strand produced in step (a); (c) amplifying the first strand cDNA obtained in step (b) with a forward primer that specifically binds to said first strand cDNA and a reverse primer, wherein the reverse primer comprises or consists of a nucleic acid sequence that corresponds to the nucleotide sequence of the oligonucleotide tag, or to a portion thereof; and (d) detecting the presence of an amplification product, wherein the presence of an amplification product obtained after reverse transcribing the RNA transcript of interest in a reaction comprising the primer of (a)(i)(1) indicates that the RNA transcript is a sense-strand RNA transcript and the presence of an amplification product obtained after reverse transcribing the RNA transcript of interest in a reaction comprising the primer of (a)(i)(2) indicates that the RNA transcript is an antisense-strand RNA transcript; and wherein the oligonucleotide tag employed in step (a)(1) has a nucleic acid sequence that is not endogenously present in the genome of the organism from which the sample has been obtained. The present invention further relates to an oligonucleotide comprising or consisting of the nucleic acid sequence shown in SEQ ID NO:1 as well as a kit comprising an oligonucleotide according to the invention and at least one of: (i) a reverse transcriptase lacking or having a reduced RNase H activity; (ii) an inhibitor of DNA-dependent DNA synthesis; (iii) RNase H; and (iv) means for amplification of cDNA.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Regulation of gene expression is a key mechanism for temporal and local directed expression of proteins and their targeted function. In addition to many ways gene transcription is controlled (e.g. transcription factors, DNA methylation, histone modification), antisense transcription is a common feature of cells and a more recently described important regulator of gene expression [1]. Antisense transcripts in mammalian cells were shown to be 72% overlapping with a part of the sense transcript and approx. 10% are directly overlapping with exons [2]. Natural antisense transcripts (NAT) can arise through independent, bidirectional or cryptic promoters and result in short and long non-coding (nc) RNAs. There are multiple mechanisms by which antisense transcripts regulate sense transcription leading to changes in gene/ protein expression. These include direct inhibition of sense transcription via antisense hybridization to RNA; induction of DNA methylation for gene silencing by antisense mediation; blockage of RNA splicing, processing, stability and miRNA binding sites via antisense/ sense hybrids; DICER targeting and processing of antisense / sense hybrids to siRNA; induction of RNA editing or changes of secondary structures through antisense/ sense hybrids and finally, inhibition of translation due to cytoplasmic antisense/ sense hybrids [3-6]. Recent publications demonstrated the importance of natural antisense RNA or NAT in the regulation of genes essential for development, differentiation and tumorigenesis. Antisense RNA can affect the function or stability of a transcribed gene (sense RNA) by: 1) inhibition of ribosome binding by forming dsRNA; 2) RNA stability through binding to sense RNA and inducing RNA cleavage, 3) induction of novel transcriptional termination through forming dsRNA and 4) as epigenetically regulators inducing chromatin remodeling and controlling gene expression [16,17].

To identify antisense transcription of a gene along with its sense transcription, different methodologies are used in the art. Besides bioinformatics methods and databases, northern blot analysis has been the most commonly used method in the past, but is not very sensitive and specific and can only detect entire transcripts, depending on the hybridization probe used. Reverse transcriptase polymerase chain reaction (RT-PCR) is another molecular method to detect NAT. This method is highly sensitive and since the amount of cDNA template produced during first strand synthesis is proportional to the amount of starting RNA, the result can be used for quantification of sense and antisense transcripts. Different companies are presently offering "First strand cDNA RT-PCR" Kits, which are based on RT-PCR including first strand cDNA synthesis with a gene specific primer. Since the sense and antisense strand are complementary, different primer for sense and antisense transcripts are employed with the aim to detect the different transcripts.

Antisense transcription analysis based on these methods is subject to certain difficulties that influence the results of strand and gene specific first strand cDNA synthesis and PCR: 1) self-priming of template RNA through secondary structures influences the specificity of cDNA synthesis and gives false positive sense and antisense results after amplification of the cDNA; and 2) retroviral reverse transcriptase enzymes have three enzymatic activities: (i) an RNA dependent DNA polymerase, (ii) a DNA dependent DNA polymerase and especially (iii) an *RNase H* activity. The *RNase H* activity of the reverse transcriptase is capable of degrading the RNA strand of a RNA:DNA hybrid during cDNA synthesis, which initiates the inadvertent second strand cDNA synthesis and, consequently, results in the unspecific and false detections of transcripts.

Accordingly, despite the fact that a lot of effort is currently being invested into the development of methods of detecting specific antisense transcripts, there is still a need to provide additional or improved approaches enabling the accurate detection of antisense transcripts present in samples of interest.

This need is addressed by the provision of the embodiments characterised in the claims.

Accordingly, the present invention relates to a method for determining the sense/antisense polarity of an RNA transcript of interest in a sample, the method comprising: (a) reverse transcribing an RNA transcript of interest present in the sample, wherein the reverse transcription reaction comprises: (i) a primer linked to an oligonucleotide tag , wherein the primer specifically binds to (1) the sense strand of the RNA transcript of interest; or (2) the antisense strand of the RNA transcript of interest; (ii) a reverse transcriptase lacking or having a reduced RNase H activity; and (iii) an inhibitor of DNA-dependent DNA synthesis; (b) removing the RNA strand from the RNA:cDNA hybrid strand produced in step (a); (c) amplifying the first strand cDNA obtained in step (b) with a forward primer that specifically binds to said first strand cDNA and a reverse primer, wherein the reverse primer comprises or consists of a nucleic acid sequence that corresponds to the nucleotide sequence of the oligonucleotide tag, or to a portion thereof; and (d) detecting the presence of an amplification product, wherein the presence of an amplification product obtained after reverse transcribing the RNA transcript of interest in a reaction comprising the primer of (a)(i)(1) indicates that the RNA transcript is a sense-strand RNA transcript and the presence of an amplification product obtained after reverse transcribing the RNA transcript of interest in a reaction comprising the primer of (a)(i)(2) indicates that the RNA transcript is an antisense-strand RNA transcript; and wherein the oligonucleotide tag employed in step (a)(1) has a nucleic acid sequence that is not endogenously present in the genome of the organism from which the sample has been obtained.

The term "determining the sense/antisense polarity of an RNA transcript", in accordance with the present invention, refers to the identification of whether an RNA transcript is a sense RNA (sRNA) transcript or an antisense RNA transcript (asRNA). As used herein, the term "sense RNA transcript" refers to mRNA, i.e. to RNA that conveys the genetic information from the DNA to the ribosome, where it is translated into proteins. The term "antisense RNA transcript", on the other hand, is used herein to refer to a single-stranded RNA that is complementary to said (full length or partial) mRNA strand transcribed within a cell.

In accordance with the present invention, an "RNA transcript of interest", also referred to herein as "target", is any RNA for which the information is desired whether it is present as the sense transcript, the antisense transcription, or both. For example, this information may be of relevance where it is known that the expression of a particular gene is regulated via the presence of its antisense transcript that inhibits the translation from the sense transcript when both are present. Such RNA transcripts of interest include, for example genes essential for development, cell cycle, proliferation and apoptosis as well as genes playing a role as oncogenes or tumor suppressors. Specific examples include, without being limiting, the transcripts of master cell regulators like p53, key genes of cell pathways like protein kinase B (AKT) or chromatin remodeling like histone acetylases, oncogenes like ras and src as well as tumor suppressors like PTEN and CD95.

The term "sample", in accordance with the present invention, refers to any sample that contains or is suspected to contain an RNA transcript of interest. Non-limiting examples of suitable samples include cells, tissues, or fluids containing RNA, such as, for example, whole blood, cerebrospinal fluid (CSF), lymph, saliva, milk, urine, or semen. The term "cells" encompasses cells obtained from a tissue, such as e.g. a biopsy or autopsy tissue as well as cells obtained from cell cultures, such as e.g. primary cell cultures or cultures of established cell lines.

The method of the present invention comprises a first step of reverse transcribing the RNA transcript of interest present in the sample. The term "reverse transcribing" is well known in the art and relates to the transcription of an RNA strand to a copy-DNA (cDNA) strand. To achieve reverse transcription, any method available in the art can be employed, provided that at least the below discussed steps and compounds are included. Such basic methods for reverse transcription are well known in the art and have been described, e.g. in Lewin's Genes IX (2013) Jones & Bartlett Publ., ISBN-13: 9781449659851 as well as the appended examples.

As a first requirement, the reverse transcription reaction comprises a primer linked to an oligonucleotide tag.

The term "primer" refers to a single stranded nucleic acid molecule that specifically hybridises to the target nucleic acid molecule and which serves as a starting point for the synthesis of a nucleic acid molecule. The requirement that the primer "hybridises specifically" means that the primer hybridises under stringent hybridisation conditions to its target. Preferably, the primer hybridise to a detectably greater degree to the target than to any other sequence (e.g., at least 2-fold over background). Preferably, the primer hybridises only to sequences that have at least 90% sequence identity, more preferably 95%, such as 98% and more preferred 100% sequence identity with the target which they are intended to bind.

In accordance with the present invention, the primer specifically binds to either the sense strand of the RNA transcript of interest; or the antisense strand of said RNA transcript of interest. In other words, the primer is not only target (gene) specific, but also strand specific. Such primers that are gene- and strand-specific are also abbreviated herein as "GSP sense" and "GSP antisense", while the term "GSP" generally refers to "gene-specific primers" herein.

Suitable primers can be designed by methods well-known in the art including, for example, publicly available programs such as primer3 (University of Massachusetts Medical School, U.S.A. (in the world wide web at frodo.wi.mit.edu/)), Primer-Blast (National Library of Medicine (NLM) web pages (in the world wide web at ncbi.nlm.nih.gov/tools/primer-blast/)), NetPrimer (PREMIER Biosoft International web pages (in the world wide web at premierbiosoft.com/netprimer/)), Primer fox (in the world wide web at primerfox.com/ by Stephan Fuchs (Ernst-Moritz-Arndt-University Greifswald)), IDT PrimerQuest (Integrated DNA Technologies (IDT) web pages (in the world wide web at eu.idtdna.com/Scitools/Applications/Primerquest/), Primo Pro (Chang Bioscience, Inc. web pages (in the world wide web at changbioscience.com/primo/primo.html)). Primers can also be designed manually, following guidelines as mentioned for example on the web pages referred to above or in the following references: Sharrocks, A.D., The design of primers for PCR, in PCR Technology, Current Innovations, Griffin, H.G., and Griffin, A.M, Ed., CRC Press, London, 1994, 5-11; Dieffenbach, C.W., Lowe, T.M.J., Dveksler, G.S., General Concepts for PCR Primer Design, in PCR Primer, A Laboratory Manual, Dieffenbach, C.W, and Dveksler, G.S., Ed., Cold Spring Harbor Laboratory Press, New York, 1995, 133-155.

The term "oligonucleotide tag", as used herein, relates to an oligonucleotide sequence that does not hybridise with the target nucleic acid molecule. The oligonucleotide tag may be of any length that ensures that its sequence is unique, i.e. that it is not present in the genome of the organism from which the sample has been obtained. Accordingly, where samples of a specific type of organism are to be analysed, it has to be ensured that the genome of this organism does not possess nucleic acid molecules that comprise the sequence or the complementary sequence of the oligonucleotide tag. Preferably, the sequence or complementary sequence of the oligonucleotide tag is absent in the human genome, more preferably it is absent in both the human and mouse genome, even more preferably it is absent in the human and rodent genome. Even more preferably, the sequence or complementary sequence of the oligonucleotide tag is absent in all mammalian genomes and most preferably it is absent in all eukaryotic genomes.

Means and methods for designing such unique sequences are well known in the art and include, without being limiting, publicly accessible programs primer design programs like "primer3plus" or "NCBI/primer-blast". Considerations when designing such sequences include, for example, (i) a length of the oligonucleotide tag that ensures that hybridisation cannot occur in the target genome (e.g. the highest hybridisation possibility in the human and mouse genome is 15 nucleotides and in the rat genome 16 nucleotides); (ii) the overall length of the final primer-oligonucleotide tag construct, which ideally should not exceed 48 to 49 nucleotides (thereby leading to an optimal length of the oligonucleotide tag shown in the examples to approx. 23 nucleotides, due to the fact that the GSP sense or GSP antisense primers employed herein and fused to the tag primer are at least 2 nucleotides longer (=25 nt)); (iii) the absence of thymidine (T), because T is more prone to mis-priming than other nucleotides; (iv) the absence of the potential to form hairpins, the absence of potential annealing with the forward primer employed in the later PCR reaction and the absence of self-annealing sites (with the allowance of one mismatched nucleotide); (v) a certain GC content, such as e.g. a GC content over 62% and not over 67%, to avoid non-specific annealing of the primer; (vi) a sufficiently high melting temperature, such as e.g. a Tm of over 60°C, because the tag primer is fused to GSP sense or GSP antisense primer and will not non-specifically hybridise during the first strand cDNA synthesis; (vii) and a direct sequence of two A, C, and/or G should be avoided.

The above parameters are used in primer design programs and the designed oligonucleotide tags are then tested against the entire human and murine genome using for example the computer program NCBI-blast (see the world wide web at blast.ncbi.nlm.nih.gov/Blast.cgi) and oligonucleotide tags that do not have a positive hybridisation against either or - preferably - both genomes are selected.

Non-limiting examples of unique sequences not present in the human or murine genome are shown in e.g. SEQ ID NO:1, as well as in SEQ ID Numbers: 2 to 6.

In accordance with the present invention, the primer is linked to said oligonucleotide tag. It will be appreciated that the oligonucleotide tag is linked via its 3' end to the 5' end of the primer. This order ensures that reverse transcription can take place by extending the 3' end of the primer sequences. The oligonucleotide tag is preferably directly linked to the primer, i.e. via a phosphodiester bond between most 3' nucleotide of the oligonucleotide tag and the most 5' nucleotide of the primer.

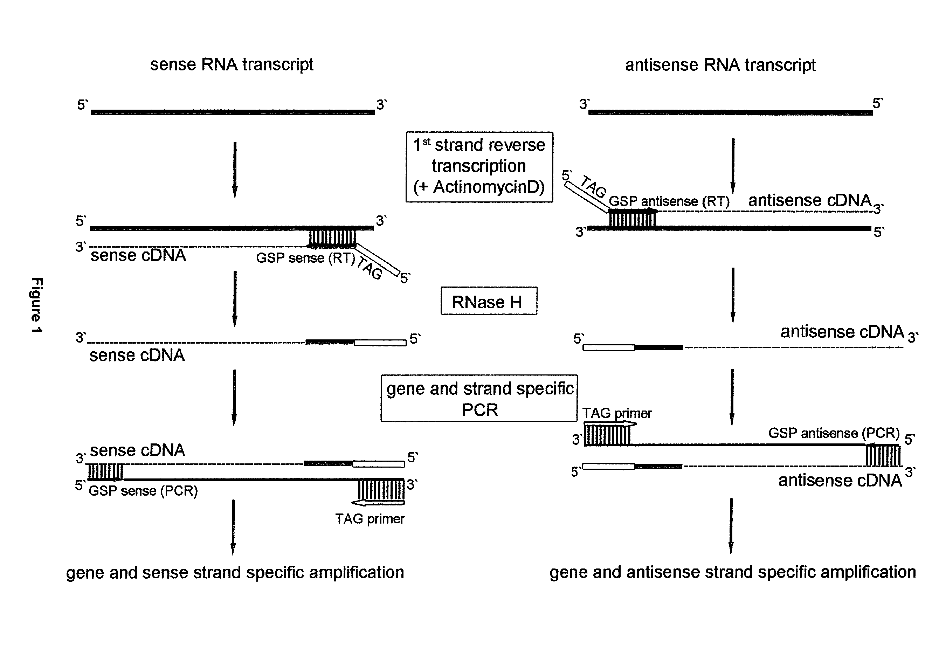

As is shown in Figure 1, the primer (see e.g. "GSP sense (RT)" in the left panel) linked to the oligonucleotide tag binds to the target RNA transcript of interest (in the left panel to the sense RNA transcript of interest). Because the oligonucleotide tag does have a complementary sequence in the target RNA transcript, it does not bind thereto. However, upon reverse transcription, the resulting first-strand cDNA transcript contains the oligonucleotide tag at its 5' end, where it serves as a primer binding site for the later amplification reaction.

As a second requirement, the reverse transcription reaction comprises a reverse transcriptase lacking or having a reduced RNase H activity.

The term "reverse transcriptase" refers to an enzyme that catalyzes the polymerisation of deoxyribonucleoside triphosphates to form primer extension products that are complementary to a ribonucleic acid template, here the RNA transcript of interest. The enzyme initiates synthesis at the 3'-end of the primer and proceeds toward the 5'-end of the template, where synthesis terminates. Examples of commonly used reverse transcriptase enzymes that convert an RNA target sequence into a complementary, copy-DNA (cDNA) sequence are avian myeloblastosis virus (AMV) reverse transcriptase, Moloney Murine Leukemia Virus (MMLV) reverse transcriptase, and Thermus thermophilus DNA polymerase, a thermostable DNA polymerase with reverse transcriptase activity marketed by Perkin Elmer. The above reverse transcriptases are mostly from retroviral origin and have a DNA-polymerase linked to a RNase H [18]. However, as mentioned above, the RNase H activity is not desired during the first strand cDNA synthesis due to the possibility of second strand artefacts, therefore reverse transcriptases with customarily inactive RNase H should be used. Especially in the HIV research inhibitors for the reverse transcriptase including the RNAse H activity are under investigation, however to date an inhibitor for solely the reverse transcriptase RNase H activity is not available. Therefore, reverse transcriptases with inactivating mutations in the RNase H domain are used for the first strand cDNA synthesis. RNase H inactivating mutations through site directed mutagenesis, especially of the MMLV reverse transcriptase are well documented, for example in [19].

Such mutated reverse transcriptases are readily available in the art, such as e.g. Superscript@ III RT (from life technologies), a reverse transcriptase from Moloney murine leukemia virus (M-ML), which has point mutations in its intrinsic RNase H region and therefore a reduced RNase H activity. Alternative examples of reverse transcriptases lacking or having a reduced RNase H activity include, without being limiting, RevertAid H Minus Reverse Transcriptase (Thermo Scientific), AccuScript (Stratagene), ProtoScript II (NEB), M-MuLV Reverse transcriptase (RNase H minus) (HyTest), KiCqStart One Step (Sigma-Aldrich), ReverTra Ace® (Toyobo), or peqGold cDNA synthesis (peqlab).

As a third requirement, the reverse transcription reaction comprises an inhibitor of DNA-dependent DNA synthesis.

Inhibitors of DNA-dependent DNA synthesis are compounds that do not interfere with RNA-dependent DNA synthesis, but block any DNA synthesis that relies on DNA as a template. Thus, the presence of a DNA-dependent DNA synthesis inhibitor serves to prevent any second strand cDNA synthesis from the first strand cDNA obtained by the reverse transcriptase reaction from the RNA transcript of interest.

Inhibitors of DNA-dependent DNA synthesis include, without being limiting, actinomycin D, hydroxy urea, alkylating agents, anthracyclines and DNA intercalating agents like cisplatin.

In a second step, the method of the present invention comprises the removal of the RNA strand from the RNA:cDNA hybrid strand produced in step (a).

Accordingly, once the RNA transcript of interest has been reverse transcribed into a cDNA copy thereof, it is removed from the reaction. To this end, any method of removing RNA from an RNA:cDNA hybrid strand can be employed, preferably, RNA-digesting enzymes, such as RNase H, are added to specifically remove the RNA from the RNA:DNA hybrid [20].

The thus obtained first strand cDNA is then amplified in a third step of the method of the present invention. To this end, amplification is carried out with two primers: (1) a forward primer that specifically binds to said first strand cDNA obtained in the second step of the method, and (2) a reverse primer that comprises or consists of a nucleic acid sequence that corresponds to the nucleotide sequence of the oligonucleotide tag, or to a portion thereof.

The first primer is a forward primer, i.e. it binds directly to the 3' end of the first strand cDNA obtained after steps (a) and (b) have been carried out. This forward primer is designed such that it hybridises specifically to the cDNA obtained from the target RNA transcript to be detected.

Upon extension of the 3' end of this gene-specific forward primer (see e.g. the "GSP sense (PCR) in the left panel of Figure 1), a second strand of cDNA is synthesised which is complementary to the first strand cDNA. The 3' end of this second strand cDNA comprises a sequence that is complementary to the oligonucleotide tag that was introduced into the first strand cDNA. Accordingly, the reverse primer, which comprises or consists of a nucleic acid sequence that corresponds to the nucleotide sequence of the oligonucleotide tag, or to a portion thereof, can now bind to the 3' end of the second strand cDNA.

In accordance with the present invention, the reverse primer may consist of a nucleic acid sequence that is identical to only a (consecutive) portion of the nucleic acid sequence of the oligonucleotide tag. It will be appreciated that in that case, said portion of the nucleic acid sequence of the oligonucleotide tag is the most 3' portion thereof, in order to ensure binding to the second strand cDNA and primer extension. In other words, where the reverse primer consist of a nucleic acid sequence that is only identical to a portion of the nucleic acid sequence of the oligonucleotide tag, it is preferred that it is identical to the first 16 consecutive nucleotides at the 3' end of the oligonucleotide tag, such as e.g. the first 17 consecutive nucleotides at the 3' end of the oligonucleotide tag, even more preferably the first 18 consecutive nucleotides, such as e.g. the first 19, the first 20, the first 21 and most preferably the first 22 consecutive nucleotides at the 3' end of the oligonucleotide tag. It is particularly preferred that the reverse primer consists of a nucleic acid sequence that is identical to the nucleic acid sequence of the oligonucleotide tag with the exception of the last one or the last two nucleotides at the 5' end of the oligonucleotide tag. Most preferably, the reverse primer consists of a nucleic acid sequence that is completely identical to the nucleic acid sequence of the oligonucleotide tag.

In as far as the reverse primer comprises (rather than consists of) the above recited sequences, it is envisaged that additional nucleotides extend over the specific sequence only at the 5' end. Preferably, no more than 20 additional nucleotides are present at the 5' end, more preferably no more than 15, such as no more than 10, more preferably no more than 9, such as no more than 8, no more than 7, no more than 6, no more than 5, no more than 4, no more than 3, no more than 2, and even more preferably no more than 1 additional nucleotide(s) is/are present at the 5' end. In those cases where such additional nucleotides are present at the 5' end of the reverse primer, it is required that the presence of these additional nucleotides does not interfere (e.g. prevent or decrease) the binding of the reverse primer to the complementary sequence of the oligonucleotide tag in the second strand cDNA. Such additional nucleotides may for example be sequences that help in e.g. the purification or detection of the subsequently obtained amplification product according to the present invention. Non-limiting examples of such a sequences include tags such as a His-tag, a Strep-tag, a GST-tag, a TAP tag, biotin, or an HA tag as well as detectable labels, including e.g. radioactive labels such as 3H, or 32P as well as fluorescent labels.

The term "amplification" is well known in the art and refers to the generation of large numbers of copies of a specific template sequence, here the first strand cDNA. This is achieved by polymerase chain reaction (PCR), and is typically carried out on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. To provide a non-limiting example, PCR can be performed as described in the appended examples. To provide a further non-limiting example, PCR can be performed e.g. in a 50 $\mu$l reaction mixture containing 5 $\mu$l of 10 x PCR buffer with 1.5 mM MgCl$_2$, 200 $\mu$M of each deoxynucleoside triphosphate, 0.5 $\mu$l of each primer (10 $\mu$M), about 10 to 100ng of template DNA and 1 to 2.5 units of Taq DNA Polymerase. The primers for the amplification may be labelled or be unlabelled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, Tth polymerase, Taq polymerase, a heat-stable DNA polymerase isolated from Thermus aquaticus, Vent, Amplitaq, Pfu and KOD. The person skilled in the art knows how to optimise PCR conditions for the amplification of specific nucleic acid molecules with primers of different length and/or nucleotide composition or how to scale down or increase the volume of the reaction mix.

Finally, in a fourth step, the method of the present invention comprises the detection of the presence of an amplification product.

Any method suitable to detect a nucleic acid molecule of interest can be employed to detect the presence of an amplification product in accordance with the present invention. Methods for determining the presence or absence of nucleic acid molecules are for example described in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989); Higgins and Hames (Eds.) "Nucleic acid hybridisation, a practical approach" IRL Press Oxford, Washington DC, (1985); Nollau et al, Clin. Chem. 43 (1997), 1114-1128; Burczak and Mardis (Ed.).

For example, the presence or absence of an amplification product can be determined by an assay based on physical separation of nucleic acid molecules, by a sequencing assay, or by a hybridisation assay.

Assays based on the physical separation of nucleic acid molecules include, without limitation, denaturing gradient gel electrophoresis (DGGE), mass-spectrometric techniques, such as e.g. MALDI-TOF, as well as HPLC. Such methods are well known in the art, see for example Petersen et al., Hum. Mutat. 20 (2002) 253-259; Hsia et al., Theor. Appl. Genet. 111 (2005) 218-225; Tost and Gut, Clin. Biochem. 35 (2005) 335-350; Palais et al., Anal. Biochem. 346 (2005) 167-175.

Examples for sequencing assays comprise without limitation approaches of sequence analysis by direct sequencing, fluorescent SSCP in an automated DNA sequencer and pyro-sequencing. These procedures are common in the art, see e.g. Adams et al. (Ed.), "Automated DNA Sequencing and Analysis", Academic Press, 1994; Alphey, "DNA Sequencing: From Experimental Methods to Bioinformatics", Springer Verlag Publishing, 1997; Ramon et al., J. Transl. Med. 1 (2003) 9; Meng et al., J. Clin. Endocrinol. Metab. 90 (2005) 3419-3422.

Examples for hybridisation assays comprise, without limitation, Northern and Southern blot assays, heteroduplex analysis, allele-specific oligonucleotide hybridisation on DNA chips, assays based on the Illumina's® technology, assays based on the BeadArray® technology and TaqMan assays, see, for example, Barnes et al., Nucleic Acids Res. 33 (2005) 5914-5923; Fan et al., Biotechniques 39 (2005) 583-588; Shen et al., Mutat. Res.-Fund. Mol. M. 573 (2005) 70-82; Steemers and Gunderson, Pharmacogenomics, 6 (2005) 777-782.

Preferably, the presence of an amplification product is determined by electrophoresis on a suitable agarose gel and visualisation of the separated nucleic acid molecules using ethidium bromide, as shown in the appended examples.

Depending on the nature of the primer used in the first step of the method of the invention (i.e. the reverse transcription step), i.e. depending on whether this primer was strand-specific for the sense or the antisense strand, it is now possible to detect the presence of either the sense RNA transcript or the antisense RNA transcript. Where a sense-strand specific primer was employed, the presence of an amplification product is indicative of the presence of the sense RNA transcript in the sample and where an antisense-strand specific primer was employed, the presence of an amplification product is indicative of the presence of the antisense RNA transcript in the sample. If two reactions are carried out in parallel, one using a primer specific for the sense strand and one using a primer specific for the antisense strand, it is possible to determine whether only a sense RNA transcript is present (and the corresponding antisense RNA transcript is absent), or whether only an antisense RNA transcript is present (and the corresponding sense RNA transcript is absent), or whether both the sense RNA transcript and the antisense RNA transcript are present (or absent).

As discussed herein above, due to various limitations encountered when employing state of the art methodologies it is presently difficult to assess with certainty whether an RNA transcript is present in a sample as the sense transcript, the antisense transcript, or both due to various limitations encountered when employing state of the art methodologies.

To circumvent these problems, a novel method called TAG-aided sense/antisense transcript detection (TASA-TD) is provided herein (see Fig. 1 for a schematic outline). In accordance with the present invention, it was surprisingly found that the combined use of three specific features can overcome the limitations of the prior art methods. These three features are (i) the incorporation of a unique oligonucleotide tag into the first strand cDNA obtained from the originally present RNA transcript; (ii) the use of a reverse transcriptase enzyme with low intrinsic RNase H activity; and (iii) the addition of an inhibitor of DNA-dependent DNA synthesis, such as e.g. actinomycin D to the reverse transcriptase reaction. The use of an incorporated TAG sequence leads to the specific amplification of first strand cDNA of interest (and no other cDNA) and a correct detection of sense and antisense transcripts, because only sense or antisense strands with an incorporated tag will be amplified during the detection PCR. Furthermore, possible self-priming through hairpins etc., which is sometimes observed with prior art methods, is prevented by the use of an oligonucleotide tag-linked primer directed against the strand-and gene-specific cDNA. Because the oligonucleotide tag sequence is not endogenously present in the genome of the organism to be analysed, it cannot self-prime. The additional use of a reverse transcriptase having a low RNase H activity and the inclusion of an inhibitor of DNA-dependent DNA synthesis further inhibit second strand cDNA synthesis during the reverse transcription [7-10], thereby avoiding artefacts.

In summary, by combining these three features, the occurrence of sense or antisense artefacts could be successfully prevented.

In a preferred embodiment of the method of the invention, the oligonucleotide tag is at least 23 nucleotides in length.

The term "at least", as used herein, refers to the specifically recited amount or number but also to more than the specifically recited amount or number. For example, the term "at least 23 nucleotides in length" encompasses also at least at least 24 nucleotides in length, at least 25 nucleotides in length, at least 30 nucleotides in length, at least 40 nucleotides in length, at least 50 nucleotides in length and so on. Furthermore, this term also encompasses exactly 23 nucleotides in length, exactly 24 nucleotides in length, exactly 25 nucleotides in length, exactly 30 nucleotides in length, exactly 40 nucleotides in length, exactly 50 nucleotides in length and so on.

A length of 23 nucleotides or more renders it possible to design an oligonucleotide tag that differs from all naturally occurring sequences of 23 consecutive nucleotides present in the human or murine genome. This enables the design of a tag, such as e.g. the tag shown in SEQ ID NO:1, that is universally usable in samples from humans or mice, without any further modifications.

In a further preferred embodiment of the method of the invention, the oligonucleotide tag has a guanine-cytosine content of at least 62%.

As is well known in the art, the guanine-cytosine content of a particular sequence influences its thermostability and, consequently, the annealing temperature of a primer binding to said sequence as well as the melting temperature of double-stranded nucleic acid molecules comprising this sequence. By ensuring that the oligonucleotide tag has a guanine-cytosine content of at least 62%, it is possible to ensure that a high melting temperature is achieved, because G and C form strong bonds. More preferably, the oligonucleotide tag has a guanine-cytosine content of at least 63%, more preferably of at least 64%, such as e.g. at least 65%. It is further preferred that the oligonucleotide tag has a guanine-cytosine content of at most 67%, to avoid the unwanted stabilisation of non-specific hybridisations. Most preferably the oligonucleotide tag has a guanine-cytosine content of 65.2%, as shown e.g. for the oligonucleotide tag represented by SEQ ID NO:1.

In another preferred embodiment of the method of the invention, the oligonucleotide tag is devoid of thymidine.

In accordance with this preferred embodiment of the method of the invention, the oligonucleotide tag only consists of adenine, guanine and cytosine because thymidine is known to results in mis-priming and slippage.

In a further preferred embodiment of the method of the invention, the oligonucleotide tag has a basic melting temperature between 60°C and 64°C.

The melting temperature (Tm) is the temperature at which a nucleic acid duplex will dissociate into its single strands. The stability of a nucleic acid duplex can be measured by its Tm. The melting temperature of a nucleic acid duplex increases both with its length, and with increasing GC content. A commonly employed formula for calculating the (Tm) is:

$$Tm = 4(G + C) + 2(A + T) \,°C.$$

The term "basic melting temperature", as used herein, relates to the temperature, at which half of a nucleic acid duplex will dissociate into its single strands. When performing PCR, a buffer or master mix is typically used, which consist of different cations and, consequently, increase the Tm of the nucleic acid duplex. This Tm is thus called "salt adjusted Tm".

In accordance with this preferred embodiment of the method of the invention, the Tm is chosen to be between 60°C and 64°C. The minimum of 60°C is due to the fact that the tag primer is fused to a GSP sense or GSP antisense primer and will not non-specifically hybridise during the first strand cDNA synthesis. On the other hand, it is advantageous that the Tm is not over 64°C (basic Tm) because otherwise the necessary annealing temperature would be too high.

In yet a further preferred embodiment of the method of the invention, the oligonucleotide tag does not form hairpins, does not self-anneal to form primer-dimers and/or does not anneal with the forward primer of step (c).

The term "hairpin", as used herein, relates to a kind of secondary structure that can occur in single-stranded nucleic acid molecule wherein when two regions of the same strand, usually complementary in nucleotide sequence when read in opposite directions, base-pair to form a double helix that ends in an unpaired loop. This structure is also referred to as "hairpin loop" or "stem-loop intra-molecular base pairing".

"Primer-dimers", in accordance with the present invention, are structures that can occur when a nucleic acid sequence, typically a primer but in accordance with the present invention also an oligonucleotide tag, comprises stretches of sequences that are complementary or reverse complementary to each other, thereby resulting in the hybridisation of one copy of the sequence (i.e. the primers or, here, the oligonucleotide tag) to a second copy thereof.

It is further preferred in accordance with this embodiment that the oligonucleotide tag does not anneal with the forward primer of step (c). In other words, the oligonucleotide tag and the forward primer to be employed in the subsequent amplification are to be designed such that they do not comprise complementary sequences that would result in hybridisation of the forward primer to the oligonucleotide tag sequence.

Means and methods to ensure that the oligonucleotide tag has the above discussed structural preferences are well known in the art and have been discussed in detail herein above. For example, commonly used primer design programmes such as e.g. "primer3plus" are available online free of charge and can be used to design oligonucleotide tag sequences that fulfil the above cited criteria. The GC and temperature preferences can be analysed with known programmes, such as e.g. BioPHP - Melting Temperature (Tm) calculation (see e.g. the world wide web a biophp.org/minitools/melting_ temperature/). Once an oligonucleotide tag sequences has been designed, it is important to assure that said tag sequence is unique in human or murine genomes, for example using NCBI-blast, as mentioned herein above.

All of the above criteria reduce the chance of the occurrence of self-priming of the oligonucleotide tag with RNA transcripts and, therefore, also minimise the chances for false-positive effects.

In another preferred embodiment of the method of the invention, the sample of interest is a murine or human sample.

The term "murine sample", as used herein, refers to any sample obtained from mice.

The term "human sample" relates to any sample obtained from humans. More specifically, the human sample is a sample obtained from a human, with the proviso that the sample is not a sample derived from a human embryo for industrial or commercial purposes.

In another preferred embodiment of the method of the invention, the oligonucleotide tag comprises or consists of the nucleic acid sequence shown in SEQ ID NO:1 or a nucleic acid sequence that has at least 90% sequence identity to the nucleic acid sequence of SEQ ID NO:1 and (i) is at least 23 nucleotides in length, (ii) has a guanine-cytosine content of at least 62%, (iii) is devoid of thymidine, (iv) has a basic melting temperature between 60°C and 64°C and (v) does not form hairpins and/or does not self-anneal to form primer-dimers. More preferably, the oligonucleotide tag comprises or consists of a nucleic acid sequence that has at least 95% sequence identity, such as at least 96% sequence identity, even more preferably at least 97% sequence identity, such as at least 98% sequence identity, even more preferably at least 99% sequence identity and even more preferably at least 99.5% sequence identity. Most preferably, the oligonucleotide tag consists of the nucleic acid sequence shown in SEQ ID NO:1.

In accordance with the present invention, the term "% sequence identity" describes the number of matches ("hits") of identical nucleotides of two or more aligned nucleic acid sequences as compared to the number of nucleotides making up the overall length of the nucleic acid sequences (or the overall compared part thereof). In other terms, using an alignment, for two or more sequences the percentage of nucleotides that are the same or similar may be determined, when the sequences are compared and aligned for maximum correspondence over a window of comparison, or over a designated region as measured using a sequence comparison algorithm as known in the art, or when manually aligned and visually inspected. Those having skill in the art know how to determine percent sequence identity between sequences using, for example, algorithms such as those based on the NCBI BLAST algorithm (Altschul et al. Nucleic Acids Res. 1997; 25(17):3389-402), CLUSTAL W computer program (Thompson et al. Nucleic Acids Res. 1994; 22(22):4673-80) or FASTA (Pearson and Lipman, Proc Natl Acad Sci U S A. 1988;85(8):2444-8), as known in the art. The NCBI BLAST algorithm is preferably employed in accordance with this invention. The BLASTN program for nucleic acid sequences uses as default a word length (W) of 11, an expectation (E) of 10, M=5, N=4, and a comparison of both strands. Preferably, the cited "% sequence identity with SEQ ID NO:1" is over the entire length of the oligonucleotide tag.

In those embodiments where the oligonucleotide tag comprises (rather than consists of) the recited sequence, additional nucleotides extend over the specific sequence either at the 5' end or the 3' end or both. Preferably, no more than 50 additional nucleotides are present at the 5' end and no more than 50 additional nucleotides are present at the 3' end. More preferably no more than 40, such as no more than 30, more preferably no more than 20, such as no more than 10, no more than 9, no more than 8, no more than 7, no more than 6, no more than 5, no more than 4, no more than 3, no more than 2 and even more preferably no more than 1 additional nucleotide(s) is/are independently present at either one or both of the 5' or 3' end. In those cases where such additional nucleotides are comprised in the oligonucleotide tag, it is preferred that the oligonucleotide tag having these additional nucleotides has at least the following features (i) has a guanine-cytosine content of at least 62%, (ii) is devoid of thymidine, (iii) has a basic melting temperature between 60°C and 64°C and (iv) does not form hairpins and/or does not self-anneal to form primer-dimers, as defined herein above.

As is shown in the appended examples, an oligonucleotide tag consisting of the nucleic acid sequence shown in SEQ ID NO:1 can successfully be employed in implementing the TASA-TD method of the present invention.

In a further preferred embodiment of the method of the invention, the reverse transcriptase lacking or having a reduced RNase H activity is a mutated Moloney murine leukemia virus (M-MLV) RNase H.

Mutated Moloney murine leukemia virus (M-MLV) RNase H is well known in the art and is readily available to the skilled person. For example, the company life technologies offers the enzyme Superscript® III RT, which has point mutations in its intrinsic RNase H region and therefore has a reduced RNase H activity. Other mutated Moloney murine leukemia virus (M-MLV) RNase H enzymes are for example RevertAid H Minus Reverse Transcriptase (Thermo Scientific), AccuScript (Stratagene), ProtoScript II (NEB), M-MuLV Reverse transcriptase (RNase H minus) (HyTest), KiCqStart One Step (Sigma-Aldrich), ReverTra Ace® (Toyobo), and peqGold cDNA synthesis (peqlab).

Preferably, the reverse transcriptase lacking or having a reduced RNase H activity is Superscript@ III RT. Superscript® III RT is commercially available from life technologies (catalogue number e.g. 11754050) and preferred amounts of Superscript@ III RT to be used in accordance with the present invention are in the range of between 75 and 125 Units, preferably between 85 and 110 Units, and most preferably 100 Units Superscript® III RT are employed in a cDNA synthesis reaction, such as e.g. a 20 μl cDNA synthesis reaction as shown in the appended examples.

In another preferred embodiment of the method of the invention, the inhibitor of DNA-dependent DNA synthesis is actinomycin D.

Actinomycin D (also known as Dactinomycin) is polypeptide antibiotic isolated from bacteria of the genus Streptomyces and has the systematic name 2-Amino-*N*,*N*'-bis[(6*S*,9*R*,10*S*,13*R*,18a*S*)-6,13-diisopropyl-2,5,9-trimethyl-1,4,7,11,14-pentaoxohexadecahydro-1*H*-pyrrolo[2,1-*i*] [1,4,7,10,13]oxatetraazacyclohexa decin-10-yl]-4,6-dimethyl-3-oxo-3*H*-phenoxazine-1,9-dicarboxamide. Actinomycin D is well known in the art, in particular for its ability to inhibit transcription by binding DNA at the transcription initiation complex and preventing elongation of RNA chain by RNA polymerase. In addition to this function, actinomycin D can also interfere with DNA replication, and thus acts as an inhibitor of DNA synthesis [7].

Actinomycin D can be obtained commercially, for example from Sigma (catalogue number: A1410) and preferred amounts of actinomycin D to be used in accordance with the present invention are in the range of between 200 and 280 ng, such as e.g. between 220 and 260 ng. Preferably, 240 ng actinomycin D are employed in a cDNA synthesis reaction, such as e.g. a 20 μl cDNA synthesis reaction as shown in the appended examples.

In yet a further preferred embodiment of the method of the invention, the removal of the RNA strand from the RNA:cDNA hybrid strands according to step (b) is carried out by addition of RNase H.

RNase H is well known in the art and can be obtained commercially, e.g. from life technologies (catalogue number for example 18021-014) and preferred amounts of RNase H to be used in accordance with the present invention are in the range of between 1.5 and 2.5 Units, such as e.g. between 1.75 and 2.25 Units; and most preferably, 2 Units RNase H are employed as shown in the appended examples.

The present invention further relates to an oligonucleotide comprising or consisting of (a) the nucleic acid sequence shown in SEQ ID NO:1; or (b) a nucleic acid sequence that has at least 90% sequence identity to the nucleic acid sequence of SEQ ID NO:1 and (i) is at least 23 nucleotides in length, (ii) has a guanine-cytosine content of at least 62%, (iii) is devoid of thymidine, (iv) has a basic melting temperature between 60°C and 64°C and (v) does not form hairpins and/or does not self-anneal to form primer-dimers.

In accordance with this embodiment of the present invention, an oligonucleotide is provided that is capable of being used as an oligonucleotide tag in the method of the present invention. The definitions provided herein above with regard to the oligonucleotide tag and its sequence and preferred features apply *mutatis mutandis* also to this embodiment.

The present invention further relates to a kit comprising an oligonucleotide of the invention and at least one of:

(i) a reverse transcriptase lacking or having a reduced RNase H activity;
(ii) an inhibitor of DNA-dependent DNA synthesis;
(iii) RNase H; and
(iv) means for amplification of cDNA.

The parts of the kit of the invention can be packaged individually in vials or other appropriate means depending on the respective ingredient or in combination in suitable containers or multi-container units. Manufacture of the kit preferably follows standard procedures which are known to the person skilled in the art. Whereas the term "kit" in its broadest sense does not require the presence of any other compounds, vials, containers and the like other than the recited components, the term "comprising", in the context of the kit of the invention, denotes that further components can be present in the kit. Non-limiting examples of such further components include preservatives, buffers for storage, enzymes etc.

The kit of the invention can be used for carrying out a method of the invention and can be, inter alia, employed in a variety of applications, e.g., in the diagnostic field, as research tool or in the therapeutic field. Accordingly, the oligonucleotide of the invention comprised in the kit is intended for use an oligonucleotide tag that can be coupled to a gene specific primer for use in the first step of the TASA-TD method of the present invention.

The definitions provided herein above with regard to the method of the invention, the oligonucleotide tag and the remaining parts of the kit, i.e. the reverse transcriptase lacking or having a reduced RNase H activity, the inhibitor of DNA-dependent DNA synthesis, the RNase H and the means for amplification of cDNA apply *mutatis mutandis* also to this embodiment.

In a preferred embodiment of the kit of the invention, the

(i) the reverse transcriptase lacking or having a reduced RNase H activity is a mutated Moloney murine leukemia virus (M-MLV) RNase H; and/or
(ii) the inhibitor of DNA-dependent DNA synthesis is actinomycin D; and/or
(iii) the means for amplification of cDNA comprise at least a primer consisting of a nucleic acid sequence that corresponds to the nucleotide sequence of the oligonucleotide tag, or to a portion thereof.

Again, all definitions of these preferred embodiments of the reverse transcriptase lacking or having a reduced RNase H, the inhibitor of DNA-dependent DNA synthesis and the means for amplification of cDNA provided herein above apply mutatis also with regard to these preferred parts of the kit of the invention.

Unless otherwise defined, all technical and scientific terms used herein have the same meaning as commonly understood by one of ordinary skill in the art to which this invention belongs. In case of conflict, the patent specification, including definitions, will prevail.

All the sequences accessible through the Database Accession numbers cited herein are within the scope of the present invention irrespective of whether the entry of the respective Accession No. is completely identical to the sequence displayed by the corresponding SEQ ID NO due to potential future updates in the database. Thus, this is to account for future corrections and modifications in the entries of GenBank, which might occur due to the continuing progress of science.

As regards the embodiments characterised in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims. To give a non-limiting example, the combination of claims 9, 8 and 5 is clearly and unambiguously envisaged in view of the claim structure. The same applies for example to the combination of claims 9 and 5, etc..

The figures show:

**Figure 1:** Schematic drawing of the TASA-TD methodology used for first strand cDNA synthesis with a gene and strand specific tag labelled primer (GSP sense/antisense (RT) TAG) and the following PCR with another gene and strand specific primer (GSP sense/antisense (PCR)) and a TAG-primer.

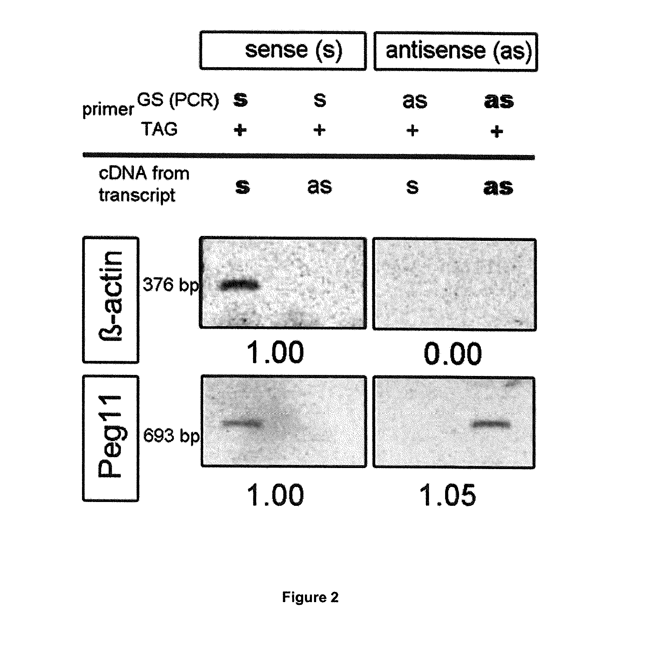

**Figure 2:** TASA-TD of ß-actin and Peg11 in mouse placenta RNA. Mouse placental RNA (E14.5) was used for TASA-TD with specific GSP sense TAG and GSP antisense TAG for first strand cDNA synthesis. Amplification of sense (s) cDNA or antisense (as) cDNA was performed with gene specific (GS) sense (s) or antisense (as) primer and TAG Primer. After electrophoresis on 1% gels, amplification products of ß-actin sense transcript and Peg11 sense and antisense transcripts were detected and quantified by ImageJ. The results indicate that ß-actin had, as previously described, no antisense transcript, whereas Peg11 had sense and antisense transcripts.

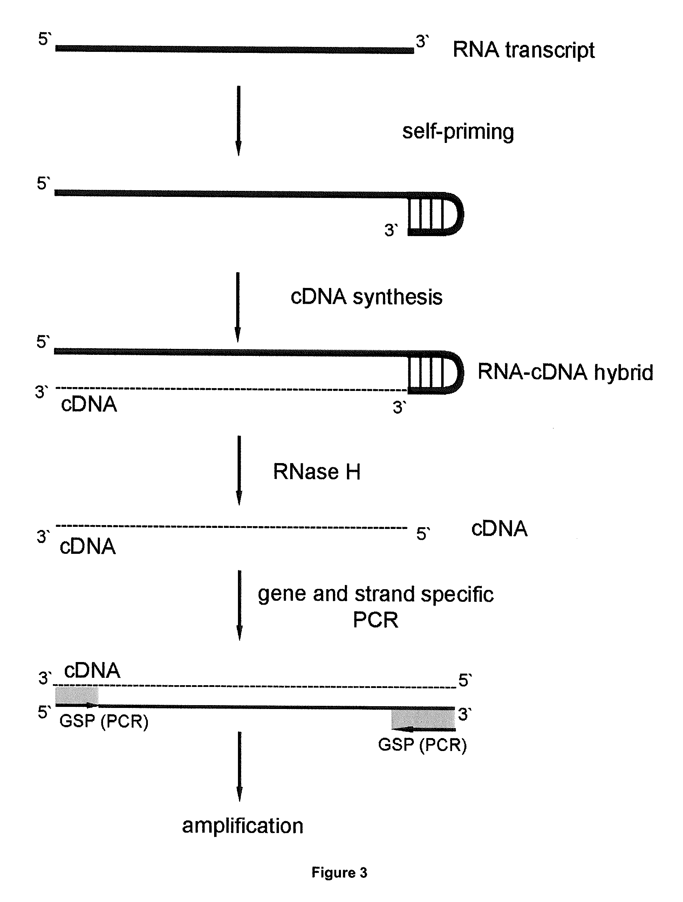

**Figure 3:** Self-priming as artefact during cDNA synthesis. Schematic drawing showing how self-priming of RNA transcripts can lead to false positive results after PCR amplification.

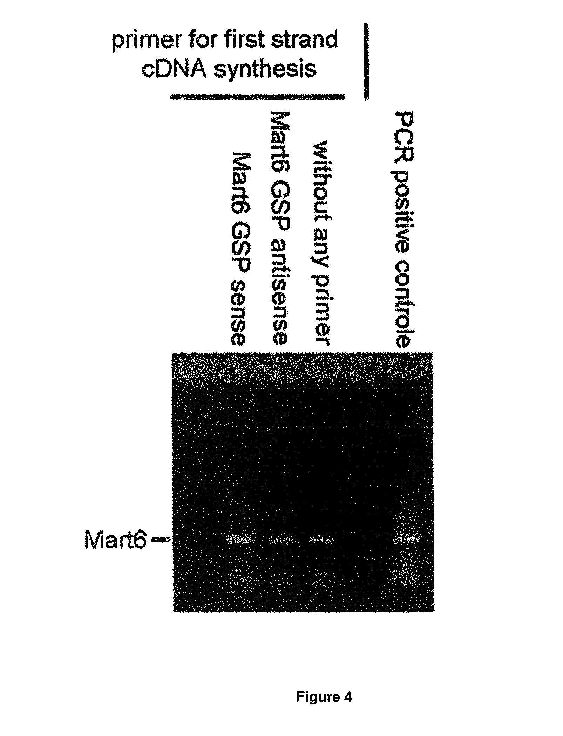

**Figure 4:** Demonstration of self-priming as artefact during TASA-TD when omitting the TAG primer. RNA of mouse placenta was used for TASA-TD with sense or antisense GSP without oligonucleotide tag or without any primer during first strand cDNA synthesis. First strand cDNA was amplified with two GSP for Mart6. In all cases, even without any primer cDNA of Mart6 were detected.

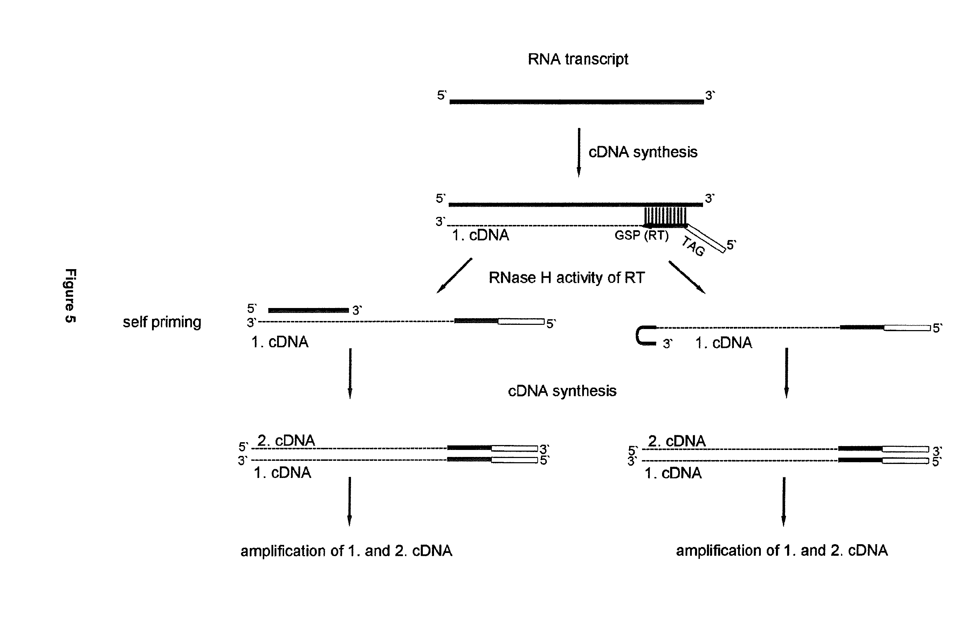

**Fig. 5:** RNase H activity of RT as artefact during TASA-TD. Schematic drawing showing that the RNase H activity of reverse transcriptase (RT) produces artefacts resulting in second cDNA synthesis and false positive results.

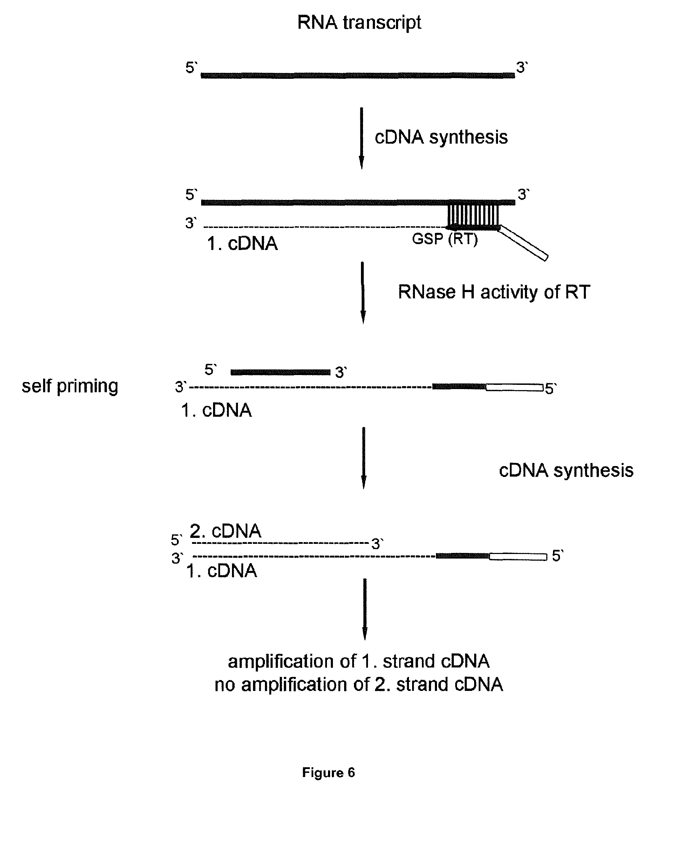

**Fig. 6:** RNase H activity and partial second strand cDNA synthesis. Schematic drawing showing the RNase H activity of reverse transcriptase (RT). Self-priming or RNA fragments can function as primer for second strand cDNA synthesis. However, if the second strand does not contain the oligonucleotide tag sequence, then the subsequent PCR amplification with TAG primer and GSP is not possible.

The examples illustrate the invention:

## Example 1: Methods and Materials

*RNA isolation*

For the establishment and verifying the TAG-aided sense/antisense transcript detection (TASA-TD) method, RNA from murine or human snap frozen placental tissues were isolated with *peqGOLD TriFast* (PeqLab) according to the manufactures protocol. After extraction, RNA was pre-treated with 40 U *DNaseI* (Roche, Germany) for 60 min at 37°C to remove any genomic DNA. After purification the RNA was resolved in DEPC treated water.

*cDNA synthesis*

Specific components from the *SuperScript III First-Strand Synthesis System for RT-PCR* (Life technologies, Germany) were implemented and adapted for the present methodology to perform reverse transcription from placental RNA. For the first strand cDNA synthesis reaction from sense and antisense RNA transcripts a specific amount of RNA, dependent on the known gene expression level, was used (e.g. 50 ng RNA for *ß-actin* and 100 or 400 ng for high to lower expressed genes, respectively). cDNA synthesis for sense and antisense transcripts were performed separately. Furthermore 1 µM *GSP-TAG* (gene and strand specific primer (GSP) ß-actin sense (5' GCTGGTCGTCGACAACGGCTC; SEQ ID NO:7), ß-actin antisense (5' CAAACATGATCTGGGTCATCTTTTC; SEQ ID NO:8), Mart6 sense (5' ATGGTCCAACCTCGGACCTCCAAAACTG; SEQ ID NO:9), Mart6 antisense (5' ATACCAGGAAGGCC ACTCTCTCGGCTTC; SEQ ID NO:10) fused to an oligonucleotide tag sequence (5' GCACACGACGAC AGACGACGCAC 3'; SEQ ID NO:1), 0.5 mM *dNTP,* 5 mM MgCl2, 10 mM *DTT,* 40 U *RNaseOUT,* 100 U *SuperScriptIII® RT* (Moloney murine leukemia virus (M-MLV), life technologies, Germany) and 240 ng *Actinomycin D* (Sigma, Germany) were added for a 20 μl reaction. RNA and primers were preheated in a 10 μl reaction at 65°C for 5 min and the reverse transcription synthesis was performed at 50°C for 50 min and terminated at 85°C for 5 min. After the cDNA synthesis was terminated, 2 U of a recombinant *RNase H* (life technologies) was added to each reaction and incubated 20 min at 37°C to ensure that the RNA of all RNA:DNA hybrids formed during the first strand RT reaction is degraded and that only single stranded DNA (the first strand cDNA of either sense or antisense RNA) is present. Due to the inactivation of the RT, no further DNA synthesis is possible. First strand cDNA mix was purified via ethanol precipitation and dissolved in 10 μl sterile water.

*Gene and strand specific PCR*

In order to amplify and detect sense transcripts, the cDNA from the first strand cDNA synthesis of sense RNA, TAG-primer and GSP-sense were used. Similarly, for the amplification and detection of antisense cDNA, TAG-primer and GSP-antisense was used. As an internal negative control, sense and antisense specific PCR was performed for both sense and antisense cDNA of *ß-actin,* which is known to have no antisense transcript [6]. PCR reactions were implemented with the *Fast-Start Taq-Polymerase Kit* (Roche). 2 μl first strand cDNA were amplified with 10 mM *dNTPs,* 5 mM TAG-Primer and 5 mM GSP-sense or -antisense and 1 U *FastStart Taq Polymerase* in a reaction of 25 μl. All cDNA products were electrophoresed on 1% agarose gels, visualized using ethidium bromide and then original tif-images were quantified using ImageJ (see the world wide web at imagej.nih.gov).

**Example 2: TAG-aided sense/antisense transcript detection TASA-TD**

In accordance with the present invention, a new approach called the TAG-aided sense/antisense transcript detection (TASA-TD) method was developed in order to identify and quantify sense and antisense transcripts. For sense and antisense RNA transcript analysis, RNA was isolated as described above. An overview of the technique is represented in Fig.1.

*The oligonucleotide tag*

One key feature of the TASA-TD is an oligonucleotide tag with a sequence not present in the mouse or human genome. The oligonucleotide tag employed herein was 23 nucleotide long and has the following sequence: 5'-GCACACGACGACAGACGACGCAC-3' (SEQ ID NO: 1). This oligonucleotide tag (TAG) can be fused to all gene specific primers (GSP) used for genes of interest by normal primer synthesis serves as the primer binding site in the subsequent detection PCR to amplify the gene- and strand-specific transcripts/cDNA. The TAG sequence was designed considering specific properties:

1) 65.2% GC content without any thymidine (T);
2) a melting temperature Tm of 62.4°C (basic) and 69.9°C salt adjusted;
3) lack of any potential hairpin formations, no potential annealing with the forward primer of the later PCR reaction and no potential self-annealing sites (with the allowance of one mismatched nucleotide). These properties of the TAG inhibit any self-priming with RNA transcripts and therefore any possible artefacts obscuring the results.

*The RT for the first strand cDNA synthesis:*

For the first strand cDNA synthesis of RNA transcripts, which is essential in order to detect sense or antisense strands, a RT with reduced *RNAse H* activity was employed, namely Superscript@ III RT (life technologies), which has point mutations in the intrinsic *RNase H* region and therefore reduced *RNase H* activity. A reduced *RNAse H* activity during the initial reverse transcription process is important to decrease the cleavage of the produced RNA:DNA hybrids into single stranded DNA, which could serve as a (unwanted) template for a second strand cDNA synthesis.

*Actinomycin D prevents second strand artefacts*

In order to completely abolish second strand syntheses artefacts, Actinomycin D (Sigma-Aldrich) was added, which inhibits DNA dependent DNA synthesis of RT. The protection of the first strand cDNA without synthesis of following cDNA strands is essential for the distinction of sense and antisense transcripts.

*The extraction of gene and strand specific cDNA and their detection*

The RT reaction was terminated by heat inactivation and the produced first strand (single) sense or antisense cDNA/RNA hybrids were subsequently digested with a recombinant *RNase H* to generate single strand cDNA. The single stranded cDNAs were purified and the gene and strand specificity detected by a PCR amplification using a 5'→ 3' GSP sense or antisense (PCR) and the 3'→ 5' TAG primer (Fig. 1). Important for the specificity of the TASA-TD method is the use of a TAG primer, which only binds to the sense or antisense cDNA of a specific gene if in the first step the corresponding TAG sequence has been introduced into the cDNA (Fig. 1). The tag sequence has been incorporated via the GSP sense or GSP antisense primer fused with the tag used during the first strand cDNA synthesis. The sense or antisense cDNA was then amplified by GSP sense or GSP antisense primer specific for the PCR reaction (without a tag fusion) and the thus produced complementary second strand was then used as a template for the TAG primer (i.e. a primer specifically binding to the reverse complement of the oligonucleotide tag). The results were then visualized with an Agarose gel and staining of the DNA.

**Example 3: Successful detection of sense/antisense transcripts using TASA-TD**

To assess the TASA-TD method, RNA from mouse placentas (E14.5) was used and TASA-TD was performed for the housekeeping-gene *ß-actin,* which has no antisense transcript [6]. As a positive antisense control *Peg11* (*paternally expressed gene* 11) was used, which is expressed in mouse placenta and in other tissues and species. Previously, antisense transcripts of *Peg11* were identified by Northern Blot analysis [11-14]. Fig.2 shows the results of TASA-TD with *ß-action* and *Peg11* after PCR amplification and electrophoresis of the PCR products on a 1% agarose gel. As expected, the result of *ß-actin* showed no antisense transcript. In contrast, *Peg11* TASA-TD showed almost equal amounts of sense and antisense transcripts (ratio using ImageJ: 1.00:1.05). As negative controls, PCR was performed of (1) sense cDNA with GSP-antisense and TAG primer, as well as of (2) antisense cDNA with GSP-sense and TAG primer. Considering the strand (sequence) specificity of sense and antisense transcripts, GSP-antisense did not amplify sense cDNA and *vice versa* (Fig. 2).

**Example 4: Possible artefacts by self-priming of the RNA is prevented using TASA-TD**

RNA transcripts regularly form secondary structures like hairpins and stem/loops because of intrinsic sequence repeats or pairing complementary sequences. These structures can induce self-priming during reverse transcription of the cDNA, which results in highly unspecific (with regard to strand-specificity) cDNA synthesis [15]. After a self-priming event, first strand cDNA of any RNA transcript can be synthesized in a RNA:DNA hybrid. After *RNase H* treatment first strand cDNA can be amplified and detected as a false positive result (Fig. 3).

To test this hypothesis, a first strand cDNA synthesis reaction was set up with all components as described above (see Materials and Methods; "cDNA synthesis"), but without any GSP or TAG primer. After *RNase H* treatment, PCR amplifications with two GSP for the gene *Mart6* were performed (Fig. 4). In parallel, first strand cDNA synthesis with GSP for sense and antisense transcripts without using the TAG primer was performed. The detection on an agarose gel showed specific bands for sense and antisense Mart6 transcripts, as well as - surprisingly - positive products without the addition of any primer. The results in Fig. 4 point to the fact that self-priming of RNA occurred and, therefore, the first strand cDNA synthesis and any conclusion from them are incorrect.

These findings show that to circumvent this problem, implementation of a TAG fused to the primer for the strand specific cDNA synthesis is an important step for ensuring specificity in the following amplification reaction. This is because although unspecific cDNA can still occur in the reverse transcription reaction, the amplification of the gene strand of interest is specific using the TAG sequence at the 5'end on the cDNA, thereby ensuring that only those strands are amplified that correspond to a true single-strand cDNA copy of the originally present RNA transcript.

**Example 5: Possible artefacts due to the (reduced) RNase H activity of the RT**

After cDNA synthesis, the first strand cDNA and the template RNA transcript form a RNA:cDNA-hybrid which normally prevents second strand cDNA synthesis. However, if the reverse transcriptase has a fully functional *RNase H* activity, degradation of the RNA transcript can occur, thereby rendering the newly synthesized first cDNA strand available as a template for second strand cDNA synthesis. To prevent such second strand cDNA synthesis during the initial first strand cDNA synthesis, use is made of a reduced *RNase H* activity of the reverse transcriptase. However, so far no reverse transcriptase with a full non-functional *RNase H* activity is available. Therefore, artefacts cannot be fully excluded even when using a reverse transcriptase with a reduced *RNase H* activity.

Possible artefacts using a reverse transcriptase (Superscript III®) despite a reduced *RNAse H* activity were tested. After first strand cDNA synthesis using all components of TASA-TD except Actinomycin D (see Materials and Methods; "cDNA synthesis"), false positive results were detected, such as abnormal antisense transcripts of ß-actin. These artefacts could only be due to the remaining activity of *RNAse H* in the Superscript III reverse transcriptase (Fig. 5). Without wishing to be bound by theory, the inventors believe that one of the following scenarios may have happened:

1) Free first strand cDNA single strands (not complexed in RNA:cDNA hybrids) have formed hairpins or loops and functioned as a template for second strand cDNA synthesis by the reverse transcriptase; 2) Released RNA of the RNA:cDNA hybrid functioned as primers for a second strand cDNA synthesis by the reverse transcriptase.

As is evident from the above, in the absence of Actinomycin D, not only RNA, but also the first strand cDNA or parts of it can be released after intrinsic *RNase H* degradation of the reverse transcriptase. The free cDNA can function as a primer itself for the second strand cDNA synthesis and produce artefacts and false positive results. Inevitably, such a mix of first and second strand cDNA leads to amplifications of false sense and antisense gene strands, as well as to false results regarding the quantity of gene expression.

Accordingly, in addition to using a reverse transcriptase with reduced *RNase H* activity, the use of Actinomycin D is imperative during RT reaction [7], in order to inhibit second strand cDNA synthesis. In the context of the present TASA-TD method, second strand cDNA products resulting from such a possible priming and self-priming of free first strand cDNA cannot be amplified due to the missing TAG sequence (Fig. 6).

## Example 5: Conclusion

The TASA-TD method presented herein is a RT-PCR technique based on a novel TAG sequence. The existing possible artefacts of general RT-PCR techniques were solved by the integration of the TAG and the inhibition of second strand cDNA synthesis by a RT with reduced *RNase H* activity and Actinomycin D. The method is easy to use, straightforward and adaptable for all genes.

## (Further) References

1. Pelechano, V. and L.M. Steinmetz, Gene regulation by antisense transcription. Nature Reviews Genetics, 2013.
2. Katayama, S., et al., Antisense transcription in the mammalian transcriptome. Science, 2005. 309(5740): p. 1564-1566.
3. Yelin, R., et al., Widespread occurrence of antisense transcription in the human genome. Nature biotechnology, 2003. 21(4): p. 379-386.
4. He, Y., et al., The antisense transcriptomes of human cells. Science, 2008. 322(5909): p. 1855-1857.
5. Faghihi, M.A. and C. Wahlestedt, Regulatory roles of natural antisense transcripts. Nature reviews Molecular cell biology, 2009. 10(9): p. 637-643.
6. Chen, J., et al., Over 20% of human transcripts might form sense-antisense pairs. Nucleic acids research, 2004. 32(16): p. 4812-4820.
7. Perocchi, F., et al., Antisense artifacts in transcriptome microarray experiments are resolved by actinomycin D. Nucleic acids research, 2007. 35(19): p. e128.
8. MULLER, W.E., R.K. ZAHN, and H.J. SEIDEL, Inhibitors acting on nucleic acid synthesis in an oncogenic RNA virus. Nature, 1971. 232(31): p. 143-145.
9. Sobell, H.M., Actinomycin and DNA transcription. Proceedings of the National Academy of Sciences, 1985. 82(16): p. 5328-5331.
10. Ruprecht, R.M., N. Goodman, and S. Spiegelman, Conditions for the selective synthesis of DNA complementary to template RNA. Biochimica et Biophysica Acta (BBA)-Nucleic Acids and Protein Synthesis, 1973. 294(2): p. 192-203.
11. Lynch, C. and M. Tristem, A Co-opted< i> gypsy</i>-type LTR-Retrotransposon Is Conserved in the Genomes of Humans, Sheep, Mice, and Rats. Current biology, 2003. 13(17): p. 1518-1523.
12. Hagan, J.P., et al., At least ten genes define the imprinted Dlk1-Dio3 cluster on mouse chromosome 12qF1. PloS one, 2009. 4(2): p. e4352.
13. Davis, E., et al., RNAi-Mediated Allelic< i> trans</i>-Interaction at the Imprinted< i> Rtl1/Peg11</i> Locus. Current Biology, 2005. 15(8): p. 743-749.
14. Bidwell, C.A., et al., Expression of PEG11 and PEG11AS transcripts in normal and callipyge sheep. BMC biology, 2004. 2(1): p. 17.
15. Beiter, T., et al., Sense or antisense? False priming reverse transcription controls are required for determining sequence orientation by reverse transcription-PCR. Analytical biochemistry, 2007. 369(2): p. 258-261.
16. Magistri M, Faghihi MA, St Laurent G 3rd, Wahlestedt C. Regulation of chromatin structure by long noncoding RNAs: focus on natural antisense transcripts. Trends Genet. 2012; 28: 389-396.
17. He Y, Meng XM, Huang C, Wu BM, Zhang L, Lv XW, Li J. Long noncoding RNAs: Novel insights into hepatocelluar carcinoma. Cancer Lett 2014; 344: 20-27
18. Champoux JJ, Schultz SJ. Ribonuclease H: properties, substrate specificity and roles in retroviral reverse transcription. FEBS J 2009; 276: 1506-1516

19. Konishi A, Hisayoshi T, Yokokawa K, Barrioluengo V, Menéndez-Arias L, Yasukawa K. Amino acid substitutions away from the RNase H catalytic site increase the thermal stability of Moloney murine leukemia virus reverse transcriptase through RNase H inactivation. Biochem Biophys Res Commun 2014; 454; 269-274

20. Wintersberger U. Ribonucleases H of retroviral and cellular origin. Pharmacol Ther. 1990;48(2):259-80.

**Claims**

1. A method for determining the sense/antisense polarity of an RNA transcript of interest in a sample, the method comprising:

(a) reverse transcribing an RNA transcript of interest present in the sample, wherein the reverse transcription reaction comprises:

(i) a primer linked to an oligonucleotide tag, wherein the primer specifically binds to

(1) the sense strand of the RNA transcript of interest; or
(2) the antisense strand of the RNA transcript of interest;

(ii) a reverse transcriptase lacking or having a reduced RNase H activity; and
(iii) an inhibitor of DNA-dependent DNA synthesis;

(b) removing the RNA strand from the RNA:cDNA hybrid strand produced in step (a);
(c) amplifying the first strand cDNA obtained in step (b) with a forward primer that specifically binds to said first strand cDNA and a reverse primer, wherein the reverse primer comprises or consists of a nucleic acid sequence that corresponds to the nucleotide sequence of the oligonucleotide tag, or to a portion thereof; and
(d) detecting the presence of an amplification product, wherein the presence of an amplification product obtained after reverse transcribing the RNA transcript of interest in a reaction comprising the primer of (a)(i)(1) indicates that the RNA transcript is a sense-strand RNA transcript and the presence of an amplification product obtained after reverse transcribing the RNA transcript of interest in a reaction comprising the primer of (a)(i)(2) indicates that the RNA transcript is an antisense-strand RNA transcript; and

wherein the oligonucleotide tag employed in step (a)(1) has a nucleic acid sequence that is not endogenously present in the genome of the organism from which the sample has been obtained.

2. The method of claim 1, wherein the oligonucleotide tag is at least 23 nucleotides in length.

3. The method of claim 1 or 2, wherein the oligonucleotide tag has a guanine-cytosine content of at least 62%.

4. The method of any one of claims 1 to 3, wherein the oligonucleotide tag is devoid of thymidine.

5. The method of any one of claims 1 to 4, wherein the oligonucleotide tag has a basic melting temperature between 60°C and 64°C.

6. The method of any one of claims 1 to 5, wherein the oligonucleotide tag does not form hairpins, does not self-anneal to form primer-dimers and/or does not anneal with the forward primer of step (c).

7. The method of any one of claims 1 to 6, wherein the sample of interest is a murine or human sample.

8. The method of any one of claims 1 to 7, wherein the oligonucleotide tag comprises or consists of the nucleic acid sequence shown in SEQ ID NO:1.

9. The method of any one of claims 1 to 8, wherein the reverse transcriptase lacking or having a reduced RNase H activity is a mutated Moloney murine leukemia virus (M-MLV) RNase H.

10. The method of any one of claims 1 to 9, wherein the inhibitor of DNA-dependent DNA synthesis is actinomycin D.

11. The method of any one of claims 1 to 10, wherein the removal of the RNA strand from the RNA:cDNA hybrid strands according to step (b) is carried out by addition of RNase H.

12. An oligonucleotide comprising or consisting of

(a) the nucleic acid sequence shown in SEQ ID NO:1; or
(b) a nucleic acid sequence that has at least 90% sequence identity to the nucleic acid sequence of SEQ ID NO:1 and

(i) is at least 23 nucleotides in length,
(ii) has a guanine-cytosine content of at least 62%,
(iii) is devoid of thymidine,
(iv) has a basic melting temperature between 60°C and 64°C and
(v) does not form hairpins and/or does not self-anneal to form primer-dimers.

13. A kit comprising an oligonucleotide according to claim 12 and at least one of:

(i) a reverse transcriptase lacking or having a reduced RNase H activity;
(ii) an inhibitor of DNA-dependent DNA synthesis;
(iii) RNase H; and
(iv) means for amplification of cDNA.

14. The kit according to claim 13, wherein

(i) the reverse transcriptase lacking or having a reduced RNase H activity is a mutated Moloney murine leukemia virus (M-MLV) RNase H; and/or
(ii) the inhibitor of DNA-dependent DNA synthesis is actinomycin D; and/or
(iii) the means for amplification of cDNA comprise at least a primer consisting of a nucleic acid sequence that corresponds to the nucleotide sequence of the oligonucleotide tag, or to a portion thereof.

Figure 1

Figure 2

**Figure 3**

Figure 4

Figure 5

RNA transcript

Figure 6

Europäisches
Patentamt

European
Patent Office

Office européen
des brevets

# EUROPEAN SEARCH REPORT

Application Number

EP 15 15 0338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HORSINGTON ET AL: "Analysis of foot-and-mouth disease virus replication using strand-specific quantitative RT-PCR", JOURNAL OF VIROLOGICAL METHODS, ELSEVIER BV, NL, vol. 144, no. 1-2, 27 July 2007 (2007-07-27), pages 149-155, XP022170253, ISSN: 0166-0934, DOI: 10.1016/J.JVIROMET.2007.05.002 * pages 150-151; figure 1; table 1 * | 1-14 | INV. C12Q1/68 |
| Y,D | F. PEROCCHI ET AL: "Antisense artifacts in transcriptome microarray experiments are resolved by actinomycin D", NUCLEIC ACIDS RESEARCH, vol. 35, no. 19, 16 October 2007 (2007-10-16), pages e128-e128, XP055195646, ISSN: 0305-1048, DOI: 10.1093/nar/gkm683 * pages 1-2,7; figure 1 * * the whole document * | 1-11,13, 14 | |
| | | | TECHNICAL FIELDS SEARCHED (IPC) |
| Y | GUO DONG-CHUAN ET AL: "Methodology for using a universal primer to label amplified DNA segments for molecular analysis", BIOTECHNOLOGY LETTERS, SPRINGER NETHERLANDS, NL, vol. 25, no. 24, 1 December 2003 (2003-12-01), pages 2079-2083, XP002501078, ISSN: 0141-5492, DOI: 10.1023/B:BILE.0000007075.24434.5E [retrieved on 2004-10-28] * page 2081; figure 1 * | 12 | C12Q |

-/--

The present search report has been drawn up for all claims

| Place of search | Date of completion of the search | Examiner |
|---|---|---|
| Munich | 17 June 2015 | Schmitt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 15 15 0338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JANNINE BROWNIE ET AL: "The elimination of primer-dimer accumulation in PCR", NUCLEIC ACIDS RESEARCH, OXFORD UNIVERSITY PRESS, GB, vol. 25, no. 16, 1 January 2007 (2007-01-01), pages 3235-3241, XP008156793, ISSN: 0305-1048 * page 3238, column 1, paragraph 2 * | 12 | |
| Y | DATABASE EMBL [Online] 10 August 2007 (2007-08-10), "rcly15b_p19.y1 cly Sus scrofa cDNA 5', mRNA sequence.", XP002741010, retrieved from EBI accession no. EM_EST:EV974624 Database accession no. EV974624 * sequence * | 12 | |
| Y | CHAOJIANG GU ET AL: "Plus and minus-stranded foot-and-mouth disease virus RNA quantified simultaneously using a novel real-time RT-PCR", VIRUS GENES, KLUWER ACADEMIC PUBLISHERS, BO, vol. 34, no. 3, 22 August 2006 (2006-08-22), pages 289-298, XP019509406, ISSN: 1572-994X * figure 1; table 1 * | 1-14 | TECHNICAL FIELDS SEARCHED (IPC) |
| Y | US 2003/104367 A1 (ROSENOW CARSTEN [US] ET AL) 5 June 2003 (2003-06-05) * claims 1,2,12,13,20,21 * | 1-11,13, 14 | |

-/--

The present search report has been drawn up for all claims

| Place of search | Date of completion of the search | Examiner |
|---|---|---|
| Munich | 17 June 2015 | Schmitt, Anja |

Europäisches
Patentamt

European
Patent Office

Office européen
des brevets

**EUROPEAN SEARCH REPORT**

Application Number

EP 15 15 0338

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 91/15601 A1 (US ARMY [US]) 17 October 1991 (1991-10-17) * claims 1,12; figures 1,5 * ----- | 1-14 | |
| T | Anonymous: "ThermoScript (TM) Reverse Transcriptase", , 1 January 2003 (2003-01-01), pages 1-4, XP055196231, Retrieved from the Internet: URL:http://tools.lifetechnologies.com/content/sfs/manuals/thermoscript_man.pdf [retrieved on 2015-06-16] * page 1 * ----- | 1-14 | |
| T | Anonymous: "SuperScript(TM) II Reverse Transcriptase", , 1 May 2010 (2010-05-01), pages 1-4, XP055196156, Retrieved from the Internet: URL:http://tools.lifetechnologies.com/content/sfs/manuals/superscriptII_pps.pdf [retrieved on 2015-06-16] * page 1 * ----- | 1-14 | **TECHNICAL FIELDS SEARCHED (IPC)** |
| T | CHRISTINE HENKE ET AL: "Selective expression of sense and antisense transcripts of the sushi-ichi-related retrotransposon - derived family during mouse placentogenesis", RETROVIROLOGY, vol. 85, no. 1, 3 February 2015 (2015-02-03), page 23, XP055196173, DOI: 10.1186/s12977-015-0138-8 * page 14, column 2, paragraph 2 - page 15, column 1, paragraph 1; figure 6A * ----- | 1-14 | |

The present search report has been drawn up for all claims

| Place of search | Date of completion of the search | Examiner |
|---|---|---|
| Munich | 17 June 2015 | Schmitt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

EP 3 042 962 A1

## ANNEX TO THE EUROPEAN SEARCH REPORT
## ON EUROPEAN PATENT APPLICATION NO.

EP 15 15 0338

17-06-2015

| Patent document cited in search report | | | Publication date | Patent family member(s) | | | Publication date |
|---|---|---|---|---|---|---|---|
| US 2003104367 | | A1 | 05-06-2003 | US | 2003104367 | A1 | 05-06-2003 |
| | | | | US | 2004126798 | A1 | 01-07-2004 |
| WO 9115601 | | A1 | 17-10-1991 | AU | 7653691 | A | 30-10-1991 |
| | | | | WO | 9115601 | A1 | 17-10-1991 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEWIN'S.** Genes IX. Jones & Bartlett Publ, 2013
- The design of primers for PCR. **SHARROCKS, A.D.** PCR Technology, Current Innovations. CRC Press, 1994, 5-11
- General Concepts for PCR Primer Design. **DIEFFEN-BACH, C.W. ; LOWE, T.M.J. ; DVEKSLER, G.S.** PCR Primer, A Laboratory Manual. Cold Spring Harbor Laboratory Press, 1995, 133-155
- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridisation, a practical approach. IRL Press Oxford, 1985
- **NOLLAU et al.** *Clin. Chem,* 1997, vol. 43, 1114-1128
- **PETERSEN et al.** *Hum. Mutat.,* 2002, vol. 20, 253-259
- **HSIA et al.** *Theor. Appl. Genet.,* 2005, vol. 111, 218-225
- **TOST ; GUT.** *Clin. Biochem.,* 2005, vol. 35, 335-350
- **PALAIS et al.** *Anal. Biochem.,* 2005, vol. 346, 167-175
- Automated DNA Sequencing and Analysis. Academic Press, 1994
- **ALPHEY.** DNA Sequencing: From Experimental Methods to Bioinformatics. Springer Verlag Publishing, 1997
- **RAMON et al.** *J. Transl. Med.,* 2003, vol. 1, 9
- **MENG et al.** *J. Clin. Endocrinol. Metab.,* 2005, vol. 90, 3419-3422
- **BARNES et al.** *Nucleic Acids Res.,* 2005, vol. 33, 5914-5923
- **FAN et al.** *Biotechniques,* 2005, vol. 39, 583-588
- **SHEN et al.** *Mutat. Res.-Fund. Mol. M.,* 2005, vol. 573, 70-82
- **STEEMERS ; GUNDERSON.** *Pharmacogenomics,* 2005, vol. 6, 777-782
- **ALTSCHUL et al.** *Nucleic Acids Res.,* 1997, vol. 25 (17), 3389-402
- **THOMPSON et al.** *Nucleic Acids Res.,* 1994, vol. 22 (22), 4673-80
- **PEARSON ; LIPMAN.** *Proc Natl Acad Sci U S A.,* 1988, vol. 85 (8), 2444-8

- **PELECHANO, V. ; L.M. STEINMETZ.** Gene regulation by antisense transcription. *Nature Reviews Genetics,* 2013
- **KATAYAMA, S. et al.** Antisense transcription in the mammalian transcriptome. *Science,* 2005, vol. 309 (5740), 1564-1566
- **YELIN, R. et al.** Widespread occurrence of antisense transcription in the human genome. *Nature biotechnology,* 2003, vol. 21 (4), 379-386
- **HE, Y. et al.** The antisense transcriptomes of human cells. *Science,* 2008, vol. 322 (5909), 1855-1857
- **FAGHIHI, M.A. ; C. WAHLESTEDT.** Regulatory roles of natural antisense transcripts. *Nature reviews Molecular cell biology,* 2009, vol. 10 (9), 637-643
- **CHEN, J. et al.** Over 20% of human transcripts might form sense-antisense pairs. *Nucleic acids research,* 2004, vol. 32 (16), 4812-4820
- **PEROCCHI, F. et al.** Antisense artifacts in transcriptome microarray experiments are resolved by actinomycin D. *Nucleic acids research,* 2007, vol. 35 (19), e128
- **MULLER, W.E. ; R.K. ZAHN ; H.J. SEIDEL.** Inhibitors acting on nucleic acid synthesis in an oncogenic RNA virus. *Nature,* 1971, vol. 232 (31), 143-145
- **SOBELL, H.M.** Actinomycin and DNA transcription. *Proceedings of the National Academy of Sciences,* 1985, vol. 82 (16), 5328-5331
- **RUPRECHT, R.M. ; N. GOODMAN ; S. SPIEGEL-MAN.** Conditions for the selective synthesis of DNA complementary to template RNA. *Biochimica et Biophysica Acta (BBA)-Nucleic Acids and Protein Synthesis,* 1973, vol. 294 (2), 192-203
- **LYNCH, C. ; M. TRISTEM.** A Co-opted< i> gypsy</i>-type LTR-Retrotransposon Is Conserved in the Genomes of Humans, Sheep, Mice, and Rats. *Current biology,* 2003, vol. 13 (17), 1518-1523
- **HAGAN, J.P. et al.** At least ten genes define the imprinted Dlk1-Dio3 cluster on mouse chromosome 12qF1. *PloS one,* 2009, vol. 4 (2), e4352
- **DAVIS, E. et al.** RNAi-Mediated Allelic< i> trans</i>-Interaction at the Imprinted< i> Rtl1/Peg11</i> Locus. *Current Biology,* 2005, vol. 15 (8), 743-749
- **BIDWELL, C.A. et al.** Expression of PEG11 and PEG11AS transcripts in normal and callipyge sheep. *BMC biology,* 2004, vol. 2 (1), 17

- **BEITER, T. et al.** Sense or antisense? False priming reverse transcription controls are required for determining sequence orientation by reverse transcription-PCR. *Analytical biochemistry,* 2007, 258-261
- **MAGISTRI M ; FAGHIHI MA ; ST LAURENT G 3RD ; WAHLESTEDT C.** Regulation of chromatin structure by long noncoding RNAs: focus on natural antisense transcripts. *Trends Genet,* 2012, vol. 28, 389-396
- **HE Y ; MENG XM ; HUANG C ; WU BM ; ZHANG L ; LV XW ; LI J.** Long noncoding RNAs: Novel insights into hepatocelluar carcinoma. *Cancer Lett,* 2014, vol. 344, 20-27
- **CHAMPOUX JJ ; SCHULTZ SJ.** Ribonuclease H: properties, substrate specificity and roles in retroviral reverse transcription. *FEBS J,* 2009, vol. 276, 1506-1516
- **KONISHI A ; HISAYOSHI T ; YOKOKAWA K ; BARRIOLUENGO V ; MENÉNDEZ-ARIAS L ; YASUKAWA K.** Amino acid substitutions away from the RNase H catalytic site increase the thermal stability of Moloney murine leukemia virus reverse transcriptase through RNase H inactivation. *Biochem Biophys Res Commun,* 2014, vol. 454, 269-274
- **WINTERSBERGER U.** Ribonucleases H of retroviral and cellular origin. *Pharmacol Ther.,* 1990, vol. 48 (2), 259-80